(19) Europäisches Patentamt
European Patent Office
Office européen des brevets

(11) **EP 3 489 349 A1**

(12) **EUROPEAN PATENT APPLICATION**
published in accordance with Art. 153(4) EPC

(43) Date of publication:
**29.05.2019 Bulletin 2019/22**

(21) Application number: **17834360.4**

(22) Date of filing: **25.07.2017**

(51) Int Cl.:
*C12N 1/00* (2006.01)    *C12M 1/00* (2006.01)
*C12M 3/00* (2006.01)    *C12N 5/00* (2006.01)

(86) International application number:
**PCT/JP2017/026938**

(87) International publication number:
**WO 2018/021358 (01.02.2018 Gazette 2018/05)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **25.07.2016 JP 2016145836**

(71) Applicant: **UBE Industries, Ltd.**
**Ube-shi, Yamaguchi 755-8633 (JP)**

(72) Inventors:
• **HAGIHARA, Masahiko**
**Ube-shi**
**Yamaguchi 755-8633 (JP)**
• **OHYA, Shusei**
**Ube-shi**
**Yamaguchi 755-8633 (JP)**

(74) Representative: **Plougmann Vingtoft a/s**
**Strandvejen 70**
**2900 Hellerup (DK)**

(54) **CELL PREPARATION METHOD, CELL CULTIVATION DEVICE, AND KIT**

(57)    The present invention relates to a cell preparation method that includes applying cells to a polyether sulfone porous film and cultivating the cells.

**EP 3 489 349 A1**

**Description**

FIELD

The present invention relates to a method for preparing cells, a cell culture apparatus and a kit.

BACKGROUND

A cell culturing method comprising the steps of applying cells to a porous polyimide film and culturing them has been reported (PTL 1).

PRIOR ART DOCUMENTS

PATENT LITERATURE

PTL 1 : WO2015/012415

DISCLOSURE OF THE INVENTION

PROBLEMS TO BE SOLVED BY THE INVENTION

An object of the present invention is to culture cells conveniently, efficiently, and stably using a porous polymer film other than polyimide porous films.

MEANS FOR SOLVING THE PROBLEMS

As a result of intensive studies in view of the above object, the present inventors have discovered that a porous polyethersulfone film can be used for culturing cells and thus have completed the present invention.
Namely, the present invention includes the following aspects.

[1] A method for preparing cells, the method comprising:
applying cells to a porous polyethersulfone film and culturing them.

[2] The method according to [1], wherein the porous polyethersulfone film is a three-layer structure porous polyethersulfone film having a surface layer A and a surface layer B, the surface layers having a plurality of pores, and a macrovoid layer sandwiched between the surface layers A and B;
wherein an average pore diameter of the pores present in the surface layer A is smaller than an average pore diameter of the pores present in the surface layer B;
wherein the macrovoid layer has a partition wall bonded to the surface layers A and B, and a plurality of macrovoids surrounded by the partition wall and the surface layers A and B; and
wherein the pores in the surface layers A and B communicate with the macrovoids.

[3] The method according to [1] or [2], the method comprising the step of:
seeding cells on the surface of the porous polyethersulfone film.

[4] The method according to [1] or [2], the method comprising the steps of:

placing a cell suspension on the dried surface of the porous polyethersulfone film;
allowing the porous polyethersulfone film to stand, or moving the porous polyethersulfone film to promote efflux of liquid, or stimulating a part of the surface to cause absorption of the cell suspension into the film; and
retaining cells in the cell suspension inside the porous polyethersulfone film, and allowing water to flow out.

[5] The method according to [1] or [2], the method comprising the steps of:

wetting one or both sides of the porous polyethersulfone film with a cell culture medium or a sterilized liquid;
loading a cell suspension into the wetted porous polyethersulfone film; and
retaining cells in the cell suspension inside the film, and allowing water to flow out.

[6] The method according to [5], wherein living cells remain within the porous polyethersulfone film, and dead cells flows out with the water.

[7] The method according to [5] or [6], wherein the sterilized liquid is a sterile water or a sterilized buffer solution.

[8] The method according to any one of [1] to [7], the method comprising the step of:
placing a cell culture medium, cells, and one or more of the porous polyethersulfone films in a cell culture vessel, wherein the porous polyethersulfone films are in a suspended state in the cell culture medium.

[9] The method according to [8], characterized in that two or more pieces of the porous polyethersulfone films are used.

[10] The method according to [8] or [9], wherein the cells spontaneously adhere to the porous polyethersulfone film.

[11] The method according to any one of [1] to [7], wherein the porous polyethersulfone film is

i) folded,
ii) wound into a roll-like shape,
iii) connected as sheets or pieces with a filamentous structure, or
iv) bound into a rope-like shape,

and suspended or fixed in a cell culture medium in a cell culture vessel.

[12] The method according to [11], wherein cells spontaneously adhere to the porous polyethersulfone film.

[13] The method according to [1] or [2], the method comprising using two or more porous polyethersulfone films are layered either above and below or left and right in the cell culture medium.

[14] The method according to [1] or [2], wherein two or more of the methods according to any one of [3] to [13] are conducted in combination.

[15] The method according to any one of [1] to [14], wherein cells grow and proliferate on the surface and the inside of a porous polyethersulfone film.

[16] The method according to any one of [1] to [15], wherein the cells are selected from the group consisting of animal cells, insect cells, plant cells, yeasts and bacteria.

[17] The method according to [16], wherein the animal cells are cells derived from an animal belonging to the vertebrate phylum.

[18] The method according to [16], wherein the bacteria are selected from the group consisting of lactic acid bacteria, Escherichia coli, Bacillus subtilis and cyanobacteria.

[19] The method according to any one of [1] to [15], wherein the cells are selected from the group consisting of pluripotent stem cells, tissue stem cells, somatic cells and germ cells.

[20] The method according to any one of [1] to [15], wherein the cells are selected from the group consisting of sarcoma cells, established cells and transformed cells.

[21] A cell culture apparatus for use in a method for preparing cells according to any one of [1] to [20], the apparatus comprising a porous polyethersulfone film.

[22] The cell culture apparatus according to [21], wherein two or more porous polyethersulfone films are layered either above and below or left and right.

[23] A kit for use in a method for preparing cells according to any one of [1] to [20], the kit comprising a porous polyethersulfone film.

EFFECTS OF THE INVENTION

According to the present invention, cells can be cultured conveniently, efficiently, and stably. In particular, the porous polyethersulfone film has a low degree of pigmentation and thus have good visibility, so that the seeding of, and the adhesion and survival behavior of cells, etc., can be visually confirmed. Furthermore, the porous polyethersulfone film has a low degree of pigmentation, and thus is excellent in designability.

BRIEF DESCRIPTION OF DRAWINGS

FIG. 1 illustrates changes over time in cell count of human skin fibroblasts cultured using porous polyethersulfone films.
FIG. 2 illustrates changes over time in cell count of human mesenchymal stem cells cultured using porous polyethersulfone films.
FIG. 3 illustrates microscopic results after induction of human mesenchymal stem cells cultured on porous polyethersulfone films into fat cells.
FIG. 4 illustrates optical micrographs when human mesenchymal stem cells cultured on porous polyethersulfone films were induced into osteoblasts, followed by mineralization induction.
FIG. 5 illustrates the results of genetic analysis after long-term culture of human mesenchymal stem cells on porous polyethersulfone films.
FIG. 6 illustrates changes over time in cell count of human osteoblasts cultured using porous polyethersulfone films.
FIG. 7 illustrates optical micrographs after mineralization induction of human osteoblasts cultured on porous polyethersulfone films.
FIG. 8] FIG. 8 illustrates changes over time in cell count of CHO DP-12 cultured using porous polyethersulfone films.
FIG. 9] FIG. 9 illustrates microscopic results of CHO DP-12 cells cultured on porous polyethersulfone films.

DESCRIPTION OF EMBODIMENTS

1. Porous polyethersulfone film to be used in the present invention

The term "porous polyethersulfone film" as used herein (hereinafter also referred to as "porous PES film") refers to a film having a plurality of pores on the surfaces, which contains polyethersulfone and typically comprises substantially polyethersulfone.

A porous polyethersulfone film to be used for the method for preparing cells of the present invention is preferably a three-layer structured porous polyethersulfone film having a surface layer A and a surface layer B having a plurality of pores, and a macrovoid layer sandwiched between the surface layer A and the surface layer B, wherein: the average pore diameter of pores existing in the surface layer A is smaller than the average pore diameter of pores existing in the surface layer B; the macrovoid layer has a partition wall bonding to the surface layers A and B and multiple macrovoids each surrounded by the partition wall and the surface layers A and B; and pores in the surface layers A and B communicate with the macrovoids. A polyethersulfone film having such a structure is hereinafter also referred to as "three-layer structured porous polyethersulfone film to be used in the invention".

The average pore diameter of pores existing in the surface layer A (hereinafter, also referred to as "surface A" or "mesh surface") in the three-layer structured porous polyethersulfone film to be used in the present invention is, but is not particularly limited to, for example, 0.01 $\mu$m or more and less than 200 $\mu$m, between 0.01 $\mu$m and 150 $\mu$m, 0.01 $\mu$m and 100 $\mu$m, 0.01 $\mu$m and 50 $\mu$m, 0.01 $\mu$m and 40 $\mu$m, 0.01 $\mu$m and 30 $\mu$m, 0.01 $\mu$m and 20 $\mu$m, or 0.01 $\mu$m and 15 $\mu$m, and preferably 0.01 $\mu$m and 15 $\mu$m.

The average pore diameter of pores existing in the surface layer B (hereinafter, may also be referred to as "surface B" or "large pore surface") in the three-layer structured porous polyethersulfone film to be used in the present invention is, but is not particularly limited to, as long as it is larger than the average pore diameter of pores existing in the surface layer A, for example, more than 5 $\mu$m and 200 $\mu$m or less, between 20 $\mu$m and 100 $\mu$m, 30 $\mu$m and 100 $\mu$m, 40 $\mu$m and 100 $\mu$m, 50 $\mu$m and 100 $\mu$m, or 60 $\mu$m and 100 $\mu$m, and preferably 20 $\mu$m and 100 $\mu$m.

The average pore diameter of the surface of the three-layer structured porous polyethersulfone film to be used in the invention can be found by measuring the pore areas of 200 or more openings based on the scanning electron micrograph of the porous film surface, and then calculating the average diameter from the average value of the pore areas according to the following Equation (1) assuming that the pore shape was perfect circle.

$$\text{Average pore diameter} = 2 \times \sqrt{(S_a / \pi)} \qquad (1)$$

(wherein Sa denotes the average value of pore areas.)

A thickness of the surface layer A or B is not particularly limited, and between, for example, 0.01 $\mu$m and 50 $\mu$m, and preferably 0.01 $\mu$m and 20 $\mu$m.

The average pore diameter of macrovoids in the film plane direction in the macrovoid layer of the three-layer structured porous polyethersulfone film to be used in the invention is not particularly limited, for example, between 10 $\mu$m and 500 $\mu$m, preferably 10 $\mu$m and 100 $\mu$m, and more preferably 10 $\mu$m and 80 $\mu$m. Furthermore, a thickness of the partition wall in the macrovoid layer is not particularly limited, for example between 0.01 $\mu$m and 50 $\mu$m, and preferably, 0.01 $\mu$m and 20 $\mu$m.

The total film thickness of the porous polyethersulfone film to be used in the invention is not particularly limited, and may be 5 $\mu$m or more, 10 $\mu$m or more, 20 $\mu$m or more or 25 $\mu$m or more, 500 $\mu$m or less, 300 $\mu$m or less, 100 $\mu$m or less, 75 $\mu$m or less or 50 $\mu$m or less. Preferably, the total film thickness is between 5 $\mu$m and 500 $\mu$m, and more preferably 25 $\mu$m and 75 $\mu$m.

The film thickness of the porous polyethersulfone film to be used in the invention can be measured using a contact type thickness meter.

The porosity of the porous polyethersulfone film to be used in the present invention is not particularly limited, and for example, 40% or more and less than 95%.

The porosity of the porous polyethersulfone film to be used in the present invention can be found by measuring the film thickness and mass of the porous film cut into a predetermined size, and then calculating with the basis mass according to the following Equation (2).

$$\text{Porosity (\%)} = (1 - w/(S \times d \times D)) \times 100 \qquad (2)$$

(wherein, S denotes the area of a porous film, d denotes the total film thickness, w denotes the mass measured, and D denotes the density of polyethersulfone. The density of polyethersulfone is designated as 1.37 g/cm$^3$.)

The porous polyethersulfone film to be used in the present invention is preferably sterilized in advance. Examples of sterilization are not particularly limited and include dry heat sterilization, steam sterilization, and sterilization using a disinfectant such as ethanol, and any sterilization such as electromagnetic wave sterilization with ultraviolet rays, a gamma ray or the like.

The porous polyethersulfone film to be used in the present invention is preferably a three-layer structured porous polyethersulfone film having the surface layer A and the surface layer B having a plurality of pores, and a macrovoid layer sandwiched between the surface layer A and the surface layer B, wherein: the average pore diameter of pores existing in the surface layer A is 0.01 $\mu$m or more and less than 200 $\mu$m, and the average pore diameter of pores existing in the surface layer B is more than 5 $\mu$m and 200 $\mu$m or less; the macrovoid layer has a partition wall bonding to the surface layers A and B, and multiple macrovoids surrounded by the partition wall and the surface layers A and B; the partition wall of the macrovoid layer, and the surface layers A and B have a thickness of 0.01 $\mu$m to 50 $\mu$m; pores in the surface layers A and B communicate with macrovoids; the total film thickness is between 5 $\mu$m and 500 $\mu$m; and the porosity is 40% or more and less than 95%.

2. Method for producing the porous polyethersulfone film to be used in the present invention

Polyethersulfone to be used as a raw material for the porous polyethersulfone film may be synthesized by a method known by persons skilled in the art. For example, polyethersulfone can be produced by a method that involves subjecting dihydric phenol, an alkali metal compound and a dihalogenodiphenyl compound to polycondensation reaction in an organic polar solvent, a method that involves synthesizing an alkali metal di-salt of dihydric phenol in advance, and then subjecting the resultant to polycondensation reaction with a dihalogenodiphenyl compound in an organic polar solvent, for example.

Examples of an alkali metal compound include alkali metal carbonate, alkali metal hydroxide, alkali metal hydride, and alkali metal alkoxide. Particularly, sodium carbonate and potassium carbonate are preferable.

Examples of a dihydric phenol compound include compounds wherein at least one of hydrogens of hydroquinone, catechol, resorcin, 4,4'-biphenol, bis(hydroxyphenyl) alkanes (for example, 2,2-bis(hydroxyphenyl)propane, and 2,2-bis(hydroxyphenyl)methane), dihydroxydiphenyl sulfones, dihydroxydiphenyl ethers, or benzene rings of these examples is substituted with a lower alkyl group such as a methyl group, an ethyl group, or a propyl group, or a lower alkoxy group such as a methoxy group or an ethoxy group. Two or more types of the above compounds can be mixed and then used as a dihydric phenol compound.

Polyethersulfone may be a commercially available product. Examples of such a commercially available product include SUMIKAEXCEL7600P and SUMIKAEXCEL5900P (both manufactured by Sumitomo Chemical Company, Limited).

Polyethersulfone has a logarithmic viscosity of, from the viewpoint of successfully forming macrovoids of the porous polyethersulfone film, preferably 0.5 or more, and more preferably 0.55 or more, from the viewpoint of production easiness for the porous polyethersulfone film, preferably 1.0 or less, more preferably 0.9 or less, further preferably 0.8 or less, and particularly preferably 0.75 or less.

Furthermore, the porous polyethersulfone film or polyethersulfone as a raw material thereof has, from the viewpoint of heat resistance and dimensional stability under high temperatures, a glass transition temperature of 200°C or higher, or no clear glass transition temperature is preferably observed.

<Production of three-layer structured porous polyethersulfone film to be used in the present invention>

The three-layer structured porous polyethersulfone film to be used the present invention can be produced by a method comprising the following steps of:

(1) flow-casting a polyethersulfone solution containing 0.3% by mass to 60% by mass of polyethersulfone having a logarithmic viscosity of 0.5 to 1.0 and 40% by mass to 99.7% by mass of an organic polar solvent to form a film, and then dipping or bringing the film into contact with a coagulating solvent containing a poor solvent or nonsolvent of polyethersulfone as an essential component, to fabricate the coagulated film having vacancies; and
(2) heat treating the coagulated film having vacancies obtained in the above step, and then coarsening the vacancies to obtain a porous polyethersulfone film, for example.

(Flow casting)

First, a polyethersulfone solution is flow-cast to form a film. The polyethersulfone solution contains preferably 0.3% by mass to 60% by mass of polyethersulfone and 40% by mass to 99.7% by mass of an organic polar solvent, more preferably 1.0% by mass to 30% by mass of polyethersulfone and 70% by mass to 99% by mass of an organic polar solvent, and further preferably 5% by mass to 15% by mass of polyethersulfone and 85% by mass to 95% by mass of an organic polar solvent.

The organic polar solvent can be selected from solvents in which polyethersulfone can be dissolved, and examples thereof include N-methyl-2-pyrrolidone (NMP), dimethyl sulfoxide, N,N-dimethylformamide, and N,N-dimethylacetamide. In view of successful formation of macrovoids, N-methyl-2-pyrrolidone is preferable.

A flow casting method is not particularly limited. For example, a polyethersulfone solution is used as a dope, the polyethersulfone solution is flow-cast onto a glass plate, a stainless plate or the like to form a film using a blade, T-die, or the like. Further, the polyethersulfone solution is intermittently or successively flow-cast onto a continuous movable belt to form a film, so that individual pieces of or a long flow-cast product can be successively produced. Such a belt may be any belt as long as it is not affected by the polyethersulfone solution and the coagulating solvent, and a belt made of metal such as stainless steel or a belt made of a resin such as polytetrafluoroethylene can be used. Moreover, the polyethersulfone solution shaped into a film through the use of T-die can be directly introduced into a coagulation bath. Furthermore, a single surface or both surfaces of a flow-cast product may be brought into contact with gasses (e.g., air and an inert gas) containing water vapor and the like, as necessary.

(Fabrication of coagulated film having vacancies)

Next, the flow-cast product is dipped or brought into contact with a coagulating solvent containing a poor solvent or nonsolvent of polyethersulfone as an essential component, polyethersulfone is precipitated to make the resultant porous, and thus a coagulated film having vacancies is fabricated. The obtained coagulated film having vacancies is washed and/or dried as necessary.

A poor solvent or nonsolvent of polyethersulfone is preferably water. The content of water in a coagulating solvent is between preferably 20% by mass and 100% by mass, more preferably 20% by mass and 80% by mass, further preferably 30% by mass and 70% by mass, and particularly preferably 40% by mass and 60% by mass. From the viewpoint of successfully forming macrovoids, a coagulating solvent containing water and an organic polar solvent is preferably used. Examples of an organic polar solvent include N-methyl-2-pyrrolidone (NMP), dimethyl sulfoxide, N,N-dimethylformamide, and N,N-dimethylacetamide. A particularly preferable organic polar solvent is N-methyl-2-pyrrolidone. When a coagulating solvent is a mixture of water and an organic polar solvent, the contents of water and the organic polar solvent in 100% by mass of the coagulating solvent are, preferably 20% by mass to 80% by mass of water and 20% by mass to 80% by mass of the organic polar solvent, more preferably 30% by mass to 70% by mass of water and 30% by mass to 70% by mass of the organic polar solvent, and further preferably 40% by mass to 60% by mass of water and 40% by mass to 60% by mass of the organic polar solvent.

The temperature of a coagulating solvent may be selected as appropriate depending on purposes, and is, for example, between -30°C and 70°C, preferably 0°C and 60°C, and further preferably 10°C and 50°C.

Subsequently, the porous polyethersulfone film is obtained by heat treating the above-obtained coagulated film having vacancies, and then coarsening the vacancies of particularly the surface layer (specifically, increasing the size of vacancies). Heat treatment involves heating a coagulated film having vacancies, within a temperature range of 80°C or higher, at a heating rate of preferably at least 10°C and more preferably 30°C/min or more, more preferably 40°C/min or more, further preferably 50°C/min or more, particularly preferably 100°C/min or more. These heating rates are advantageous in successful coarsening of vacancies. The heating rate can be, from the viewpoint of stable formation of macrovoids in a desired shape, preferably 300°C/min or less, and more preferably 270°C/min or less.

With regard to successful coarsening of vacancies, the temperature is increased to preferably the glass transition temperature or higher of polyethersulfone, or preferably 240°C or higher, or 250°C or higher, or 260°C or higher. In the meantime, from the viewpoint of preventing the decomposition of polyethersulfone during heat treatment, the temperature is increased to preferably 350°C or lower, or 300°C or lower.

In the porous polyethersulfone film, porosity, film thickness, average pore diameter, and maximum pore diameter, etc., can be adjusted through appropriate selection of a polymer type, the polymer concentration of a polymer solution, viscosity and solvent species, coagulation conditions (e.g., control of solvent substitution rate, temperature, and coagulating solvent species), and the like to be employed.

In the porous polyethersulfone film, the surface treatment of the film may be performed by performing corona discharge treatment, plasma discharge treatment such as low-temperature plasma discharge or atmospheric plasma discharge, chemical etching or the like for at least a single surface thereof according to the purpose, for example. Moreover, the surface layer A and/or B may be subjected to facing and then used. The material permeability, surface pore diameter, and wettability of the film can be controlled by these treatments.

3. Method for preparing cells of the present invention

The method for preparing cells of the present invention comprises applying cells to the porous polyethersulfone film and culturing cells. The method of the present invention comprises applying cells to the porous polyethersulfone film, and then culturing cells on the surface or in the interior of the porous polyethersulfone film.

(1) Application of cells to porous polyethersulfone film

There are no particular restrictions on the specific steps for application of the cells to the porous polyethersulfone film. It is possible to carry out the steps described throughout the present specification, or to employ any desired method suited for applying cells to a film-like support. Application of cells to the porous polyethersulfone film in the method of the present invention includes, but is not limited to, the following modes:

(A) a mode including a step of seeding cells on the surface of the porous polyethersulfone film;

(B) a mode including a step of placing a cell suspension on the dried surface of the porous polyethersulfone film, allowing it to stand, or moving the porous polyethersulfone film to promote efflux of the liquid, or stimulating part of the surface to cause absorption of the cell suspension into the film, and retaining the cells in the cell suspension inside the porous polyethersulfone film and allowing the water to flow out; and

(C) a mode including a step of wetting one or both sides of the porous polyethersulfone film with a cell culture medium solution or a sterilized liquid, loading a cell suspension into the wetted porous polyethersulfone film, and retaining the cells in the cell suspension inside the porous polyethersulfone film and allowing the water to flow out.

Mode (A) includes a step of directly seeding cells or a cell mass on the surface of a porous polyethersulfone film. Alternatively, it includes a mode of placing a porous polyethersulfone film in a cell suspension and wetting the cell culture solution from the surface of the film.

Cells seeded on the surface of a porous polyethersulfone film adhere to the porous polyethersulfone film and infiltrate into the interiors of the pores. Preferably, the cells adhere spontaneously to the porous polyethersulfone film without applying any particular exterior physical or chemical force. The cells that have been seeded on the surface of the porous polyethersulfone film can stably grow and proliferate on the surface and/or in the interior of the film. The cells may be in a variety of different forms, depending on the location of the film used for growth and proliferation.

For mode (B), a cell suspension is placed on the dried surface of a porous polyethersulfone film. The porous polyethersulfone film is allowed to stand, or the porous polyethersulfone film is moved to promote efflux of the liquid, or part of the surface is stimulated to cause absorption of the cell suspension into the film, so that the cell suspension permeates into the film. While it is not our intention to be constrained by theory, this is believed to be due to the properties of each surface forms of the porous polyethersulfone film. According to this mode, the cells are absorbed and seeded in the locations of the film where the cell suspension has been loaded.

Alternatively, as according to mode (C), after all or a portion of one or both sides of the porous polyethersulfone film has been wetted with the cell culture solution or sterilized liquid, the cell suspension may be loaded into the wetted porous polyethersulfone film. This will significantly increase the transit rate of the cell suspension.

For example, a method of wetting a portion of the film edges for the main purpose of preventing fly loss of the film (referred to as the "single-point wetting method" hereinbelow) can be used. This method is nearly the same as the dry method (mode (B)) in which the film is not essentially wetted. However, it is possible that cell solution permeation through the film is more rapid at the small wetted portions. A method in which all of one or both sides of the porous polyethersulfone film that have been thoroughly wetted (referred to as "wet film" hereinbelow) is loaded with a cell suspension (referred to as the "wet film method" hereinbelow) can be also used. In this case, the entire porous polyethersulfone film has a greatly increased transit rate for the cell suspension.

According to modes (B) and (C), the cells in the cell suspension are retained in the porous polyethersulfone film, while the water flows out. This allows treatment such as increasing the concentration of cells in the cell suspension and flowing out of unwanted non-cellular components together with the water.

Mode (A) will also be referred to as "natural seeding", and modes (B) and (C) as "suction seeding".

Preferably, but not restrictively, the viable cells are selectively retained in the porous polyethersulfone film. Thus, according to a preferred mode of the invention, the viable cells are retained in the porous polyethersulfone film, and the dead cells preferentially flow out together with the water.

The sterilized liquid used for mode (C) is not particularly restricted, and may be a sterilized buffering solution or sterilized water. A buffering solution may be, for example, (+) or (-) Dulbecco's PBS, or (+) or (-) Hank's Balanced Salt Solution. Examples of buffering solutions are listed in Table 1 below.

[Table 1]

| Component | Concentration (mmol/L) | Concentration (g/L) |
|---|---|---|
| NaCl | 137 | 8.00 |
| KCl | 2.7 | 0.20 |
| $Na_2HPO_4$ | 10 | 1.44 |
| $KH_2PO_4$ | 1.76 | 0.24 |
| pH(-) | 7.4 | 7.4 |

Application of cells to porous polyethersulfone film in the method of the present invention further includes a mode of adding adhesive cells in a floating (suspended) state as a suspension together with a porous polyethersulfone film, to adhere the cells with the film (entangling). For example, for application of the cells to the porous polyethersulfone film in the cell culturing method of the invention, the cell culture medium, the cells and one or more of the porous polyethersulfone films may be placed in the cell culturing vessel. When the cell culture medium is a liquid, the porous polyethersulfone film is in a floating (suspended) state in the cell culture medium. The cells can adhere to the porous polyethersulfone film due to the properties of the porous polyethersulfone film. Thus, even with cells that are not suited for natural suspension culture, the porous polyethersulfone film allows culturing in a floating state in the cell culture medium. The cells preferably spontaneously adhere to the porous polyethersulfone film. Here, "adhere spontaneously" means that the cells are retained on the surface or in the interior of the porous polyethersulfone film without applying any particular exterior physical or chemical force.

Cell culturing can be classified into culturing where the cultured cells are adhesion culture-type cells or suspension culture-type cells, depending on the state in the cell culture. Adhesion culture-type cells are cultured cells that adhere and grow on a culturing vessel, with the medium being exchanged at the time of subculture. Suspension culture-type cells are cultured cells that grow in a suspended state in a medium, and generally the medium is not exchanged at the time of subculture but dilution culture is carried out. Because suspension culture allows culturing in a suspended state, i.e. in a liquid, mass culturing becomes possible, and because it is three-dimensional culturing, unlike with adhering cells that grow only on the culturing vessel surface, the advantage of increased culturable cell count per unit space is afforded.

According to the invention, in conceptual terms, there is provided a method in which it is possible to grow cells in a form similar to suspension culture without being limited to the cell type, so that cells can be conveniently cultured in large amounts. According to the cell culture method of the invention, when the porous polyethersulfone film is used in a state suspended in the cell culture medium, two or more fragments of the porous polyethersulfone film may be used. Since the porous polyethersulfone film is a flexible thin-film, using such fragments that are suspended in the culture solution, for example, allows a porous polyethersulfone film with a large surface area to be added into a fixed volume of cell culture medium. In the case of normal culturing, the container base area constitutes the area limit in which cell culture can be accomplished, but with cell culturing using the porous polyethersulfone film of the invention, all of the large surface area of the previously added porous polyethersulfone film constitutes area in which cell culturing can be accomplished. The porous polyethersulfone film allows the cell culture solution to pass through, allowing supply of nutrients, oxygen and the like even into the folded film, for example.

The sizes and shapes of the porous polyethersulfone film fragments are not particularly restricted. The shapes may be as desired, such as circular, elliptical, quadrilateral, triangular, polygonal or string-like. This includes, for example, quadrilaterals (square, rectangular or the like) and triangular shapes with side lengths of about 0.1 mm to about 20 mm, preferably about 0.2 mm to about 10 mm and more preferably about 1 mm to about 5 mm. Alternatively, for example, they may be circular, with diameters of preferably about 0.1 mm to about 20 mm and more preferably about 0.5 mm to about 10 mm. Dispersing the fragments in the liquid results in a form similar to a suspension culture.

Because the porous polyethersulfone film of the invention is flexible, it can be used with varying shapes. Instead of a flat form, the porous polyethersulfone film can also be used by working into a three-dimensional shape. For example, the porous polyethersulfone film may be: i) folded, ii) wound into a roll, iii) connected as sheets or fragments by a filamentous structure, or iv) bound into a rope, for suspension or fixing in the cell culture medium in the cell culturing vessel. By forming it into shapes such as i) to iv), it is possible to place a large amount of porous polyethersulfone film into a fixed volume of cell culture medium, similar to using fragments. Furthermore, since each fragment can be treated as an aggregate, it is possible to aggregate and move the cell masses, for overall high applicability.

With the same concept as fragment aggregates, two or more porous polyethersulfone films may be used in a layered form either above and below or left and right in the cell culture medium. Layering includes a mode in which portions of the porous polyethersulfone films overlap. Layered culturing allows culturing of cells at high density in a narrow space. It is also possible to further layer a film on a film on which cells are already growing, setting it to create a multilayer of different cell types. This may also be used for drug development, including verification of intercellular interaction in a three-dimensional environment, or in a non-stress cell culture method. The number of layered porous polyethersulfone films is not particularly restricted.

Two or even more forms of the cell culturing method of the invention described above may be used in combination. For example, using any of the methods of modes (A) to (C), first the cells may be applied to the porous polyethersulfone film and then the cell-adhered porous polyethersulfone film may be used for suspension culture. Alternatively, the step of application to the porous polyethersulfone film may be a combination of two or more of the methods of any of modes (A) to (C).

In the method of the invention, preferably the cells grow and proliferate on the surface or in the interior of the porous polyethersulfone film. No reports exist disclosing growth and proliferation of cells inside a three-dimensional structure. By utilization of a porous polyethersulfone film according to the invention, it is possible to accomplish continuous three-dimensional culturing of cells. While not restrictive, the method of the invention carries out continuous growth of cells for 2 days or longer, more preferably 4 days or longer and even more preferably 6 days or longer.

(2) Cells used in the present invention

There are no particular restrictions on the type of cells that can be utilized for the method of the invention, and it may be used for growth of any type of cells.

For example, the cells may be selected from the group consisting of animal cells, insect cells, plant cells, yeast cells and bacteria. Animal cells are largely divided into cells from animals belonging to the subphylum Vertebrata, and cells from non-vertebrates (animals other than animals belonging to the subphylum Vertebrata). There are no particular restrictions on the source of the animal cells, for the purpose of the present specification. Preferably, they are cells from an animal belonging to the subphylum Vertebrata. The subphylum Vertebrata includes the superclass Agnatha and the superclass Gnathostomata, the superclass Gnathostomata including the class Mammalia, the class Aves, the class Amphibia and the class Reptilia. Preferably, they are cells from an animal belonging to the class Mammalia, generally known as mammals. Mammals are not particularly restricted but include, preferably, mice, rats, humans, monkeys, pigs, dogs, sheep and goats.

There are also no particular restrictions on sources of plant cells, for the purpose of the present specification. Suitable cells are from plants including bryophytes, pteridophytes and spermatophytes.

Plants from which spermatophyte cells are derived include both monocotyledons and dicotyledons. While not restrictive, monocotyledons include Orchidaceae plants, Poaceae plants (rice, corn, barley, wheat, sorghum and the like) and Cyperaceae plants. Dicotyledons include plants belonging to many subclasses including the subclass Chrysanthemum, the subclass Magnoliidae and the subclass Rosidae.

Algae may be considered cell-derived organisms. These include different groups, from the eubacteria Cyanobacteria (blue-green algae), to eukaryotic monocellular organisms (diatoms, yellow-green algae, dinoflagellates and the like) and multicellular marine algae (red algae, brown algae and green algae).

There are no particular limitations on the types of archaebacteria or bacteria for the purpose of the present specification. Archaebacteria are composed of groups comprising methanogenic bacteria, extreme halophilic bacteria, thermophilic acidophilic bacteria, hyperthermophilic bacteria and the like. Bacteria are selected from the group consisting of, for example, lactic acid bacteria, *E. coli, Bacillus subtilis* and cyanobacteria.

The types of animal cells or plant cells that may be used for the method of the invention are not particularly restricted, but are preferably selected from the group consisting of pluripotent stem cells, tissue stem cells, somatic cells and germ cells.

The term "pluripotent stem cells", for the purpose of the invention, is intended as a comprehensive term for stem cells having the ability to differentiate into cells of a variety of tissues (pluripotent differentiating power). While not restrictive, pluripotent stem cells include embryonic stem cells (ES cells), induced pluripotent stem cells (iPS cells), embryonic germ cells (EG cells) and germ stem cells (GS cells). They are preferably ES cells or iPS cells. Particularly preferred are iPS cells, which are free of ethical problems, for example. The pluripotent stem cells used may be any publicly known ones, and for example, the pluripotent stem cells described in WO2009/123349 (PCT/JP2009/057041) may be used.

The term "tissue stem cells" refers to stem cells that are cells lines capable of differentiation but only to limited specific tissues, though having the ability to differentiate into a variety of cell types (pluripotent differentiating power). For example, hematopoietic stem cells in the bone marrow are the source of blood cells, while neural stem cells differentiate into neurons. Additional types include hepatic stem cells from which the liver is formed and skin stem cells that form skin tissue. Preferably, the tissue stem cells are selected from among mesenchymal stem cells, hepatic stem cells, pancreatic stem cells, neural stem cells, skin stem cells and hematopoietic stem cells.

The term "somatic cells" refers to cells other than germ cells, among the cells composing a multicellular organism. With sexual reproduction, these are not passed on to the next generation. Preferably, the somatic cells are selected from among hepatocytes, pancreatic cells, muscle cells, bone cells, osteoblasts, osteoclasts, chondrocytes, adipocytes, skin cells, fibroblasts, pancreatic cells, renal cells and lung cells, or blood cells such as lymphocytes, erythrocytes, leukocytes, monocytes, macrophages or megakaryocytes.

The term "germ cells" refers to cells having the role of passing on genetic information to the succeeding generation in reproduction. These include, for example, gametes for sexual reproduction, i.e. the ova, egg cells, sperm, sperm cells, and spores for asexual reproduction.

The cells may also be selected from the group consisting of sarcoma cells, established cell lines and transformants. The term "sarcoma" refers to cancer occurring in non-epithelial cell-derived connective tissue cells, such as the bone, cartilage, fat, muscle or blood, and includes soft sarcomas, malignant bone tumors and the like. Sarcoma cells are cells derived from sarcoma. The term "established cell line" refers to cultured cells that are maintained *in vitro* for long periods and reach a stabilized character and can be semi-permanently subcultured. Cell lines derived from various tissues of various species including humans exist, such as PC12 cells (from rat adrenal medulla), CHO cells (from Chinese hamster ovary), HEK293 cells (from human embryonic kidney), HL-60 cells (from human leukocytes), HeLa cells (from human cervical cancer), Vero cells (from African green monkey kidney epithelial cells), MDCK cells (from canine kidney renal tubular epithelial cells) and HepG2 cells (from human hepatic carcinoma). The term "transformants" refers to cells with an altered genetic nature by extracellularly introduced nucleic acid (DNA and the like). Suitable methods are known for transformation of animal cells, plant cells and bacteria.

(3) Cell culture system and cell culture conditions

In the method of the present invention, a cell culture system and cell culture conditions can be determined as appropriate according to cell types, etc. Culturing methods appropriate for each type of cells including animal cells, plant cells, and bacteria are known, and persons skilled in the art can culture cells on the porous polyethersulfone film using any known method. A cell culture medium can also be prepared as appropriate according to cell types.

Cell culturing methods and cell culture media for animal cells are described in a catalog of cell culture medium of Lonza, for example. Cell culturing methods and cell culture media for plant cells are described in the plant tissue medium series of WAKO, for example. Cell culturing methods and cell culture media for bacteria are described in a catalog of media for general bacteria of Becton, Dickinson and Company, for example. A cell culture medium to be used for the method of the present invention may be in any form of liquid medium, semisolid medium, and solid medium, for example. Moreover, a liquid medium in the form of droplets may be sprayed into a cell culturing vessel, thereby bringing the medium into contact with the porous polyethersulfone film supporting cells.

When cells are cultured using the porous polyethersulfone film, cells may be allowed to coexist with another suspended culture carrier such as a microcarrier or a cellulose sponge.

In the method of the present invention, the shape, the scale and the like of a system to be used for culturing are not particularly limited, and a petri dish, a flask, a plastic bag, a test tube and even a large tank for culturing cells can be used as appropriate. Examples thereof include a cell culture dish of BD Falcon and a Nunc Cell Factory of Thermo Fisher Scientific K.K. In addition, the use of the porous polyethersulfone film in the present invention makes it possible to culture even cells, the suspension culture of which has been impossible, using an apparatus for suspension culture under conditions analogous to suspension culture conditions. As an apparatus for suspension culture, for example, a spinner flask or an apparatus for spinning culture etc., of Corning Inc., can be used. Moreover, as an environment where similar functions can be realized, a hollow fiber cell culture system such as a FiberCell (registered trademark) System of VERITAS can also be used.

Culturing in the method of the present invention can also be performed using a model wherein a continuous circulation or open-type apparatus for continuously adding medium onto the porous polyethersulfone film and then recovering the medium is used, so as to expose the porous polyethersulfone film sheet in air.

In the present invention, cells may be cultured in a system whereby a cell culture medium is continuously or intermittently fed from a cell culture medium-feeding means installed outside the cell culturing vessel into the cell culturing vessel. At this time, this can be a system whereby the cell culture medium circulates between the cell culture medium-feeding means and the cell culturing vessel.

When cells are cultured in a system whereby a cell culture medium is continuously or intermittently fed from a cell culture medium-feeding means installed outside a cell culturing vessel into the cell culturing vessel, this system may be a cell culture apparatus comprising a culturing unit that is a cell culturing vessel and a medium feeding unit that is a cell culture medium-feeding means, wherein:

the culturing unit accommodates one or a plurality of porous polyethersulfone films for carrying cells, and is provided with a medium feed port and a medium discharge port; and

the medium feeding unit is provided with a medium container, a medium feeding line, and a liquid feed pump for continuously or intermittently feeding medium via the medium feeding line, wherein a first edge of the medium feeding line contacts medium within the medium container, a second edge of the medium feeding line communicates with the interior of the culturing unit via the medium feed port of the culturing unit.

Furthermore, in the above cell culture apparatus, the culturing unit may be a culturing unit not provided with an air supply port, an air exhaust port, and an oxygen exchange membrane, and furthermore, may be a culturing unit provided with an air supply port and an air exhaust port, or an oxygen exchange membrane. Even when the culturing unit is not provided with an air supply port and an air exhaust port, as well as an oxygen exchange membrane, oxygen and the like required for culturing cells are sufficiently supplied to cells through medium. Furthermore, in the above cell culture apparatus, the culturing unit may be further provided with a medium discharging line, wherein a first edge of the medium discharging line is connected to a medium container, and a second edge of the medium discharging line communicates with the interior of the culturing unit via the medium discharge port of the culturing unit, and thus medium can circulate between the medium feeding unit and the culturing unit.

4. Cell culture apparatus of the present invention

The present invention further relates to a cell culture apparatus to be used for the preparation method of the present invention, which contains the porous polyethersulfone film. In the cell culture apparatus of the present invention, the porous polyethersulfone film may be used in a state of being fixed, or in a state of being suspended in a cell culture medium. In the cell culture apparatus, two or more porous polyethersulfone film s may be layered either vertically or laterally.

5. Kit of the present invention

The present invention also relates to a kit for use in the method for preparing cells, the kit comprising a porous polyethersulfone film.

The kit of the invention may comprise constituent elements necessary for cell culturing in addition to the porous polyethersulfone film, as appropriate. This comprises, for example, the cells applied to the porous polyethersulfone film, the cell culture medium, the cell culturing apparatus and the instruction manual for the kit.

While not restrictive, one aspect includes a package containing either one or a plurality of sterilized porous polyethersulfone films stored in a transparent pouch, in a form allowing their use for cell culturing, or a kit having sterile liquid encapsulated together with a porous polyethersulfone film in the same pouch, in the form of an integrated film/liquid allowing efficient suction seeding.

EXAMPLES

The present invention is further specifically described with reference to the following examples, however, the scope of the present invention is naturally not limited by these examples.

Example 1

Changes over time in cell count of human skin fibroblasts cultured using porous polyethersulfone films

(1) Preparation of porous polyethersulfone films 1 and 2

Polyethersulfone (SUMIKAEXCEL7600P: Sumitomo Chemical Company, Limited, logarithmic viscosity: 0.701) was weighed and then added into a 500-ml separable flask using N-methyl-2-pyrrolidone (NMP) as a solvent in such a manner that the polymer concentration was 10% by mass. Subsequently, the flask was covered with a separable cover fitted with a stirring blade, a nitrogen introducing pipe, and an exhaust pipe, and then the solution was stirred for 24 hours while keeping the temperature at 50°C with the use of an oil bath, so that a viscous and clear liquid, a polyethersulfone solution was obtained.

A 20-cm square stainless substrate having a mirror-polished surface was coated uniformly with the above-prepared polyethersulfone solution by flow casting at room temperature using a tabletop automatic coater in such a manner that the thickness was between about 100 $\mu$m and 300 $\mu$m. Subsequently, the solution was left to stand for 90 seconds in air at a temperature of 23°C and with humidity of 40%, and then the substrate was entirely placed in a mixed coagulation bath containing an aqueous solution having an NMP concentration of 40% by mass. After placement, the substrate was left to stand for 10 minutes, so as to precipitate a polyethersulfone film on the substrate. Thereafter, the substrate was removed from the bath, the polyethersulfone film precipitated on the substrate was peeled off, and then dipped in pure water for 5 minutes, thereby obtaining a polyethersulfone film. The polyethersulfone film was dried in air at a temperature of 23°C and with humidity of 40%, fitted to a 10-cm square pin tenter, and then set in an electric furnace. This was heated up to 80°C at a heating rate of about 10°C/min, heated to 270°C at a heating rate of 10°C/min, and then kept as such for 3 minutes. Heat treatment was performed according to this temperature profile, thereby preparing a porous polyethersulfone film 1 (total film thickness of 25 $\mu$m) and a porous polyethersulfone film 2 (total film thickness of 50 $\mu$m) having different thicknesses. The porous polyethersulfone films 1 and 2 may also be referred to as "porous PES film 1" and "porous PES film 2", respectively as follows. Both films are three-layer structured porous polyethersulfone films each having a surface layer A and a surface layer B having multiple pores, and a macrovoid layer sandwiched between these surface layer A and surface layer B.

The average pore diameter of pores existing in the surface layer A of the porous PES film 1 was 14 $\mu$m, the average pore diameter of pores existing in the surface layer B of the same was 27 $\mu$m, and the porosity was 58%.

The average pore diameter of pores existing in the surface layer A of the porous PES film 2 was 16 $\mu$m, the average pore diameter of pores existing in the surface layer B of the same was 31 $\mu$m, and the porosity was 61%.

(2) Culture of human skin fibroblasts using porous PES films 1 and 2

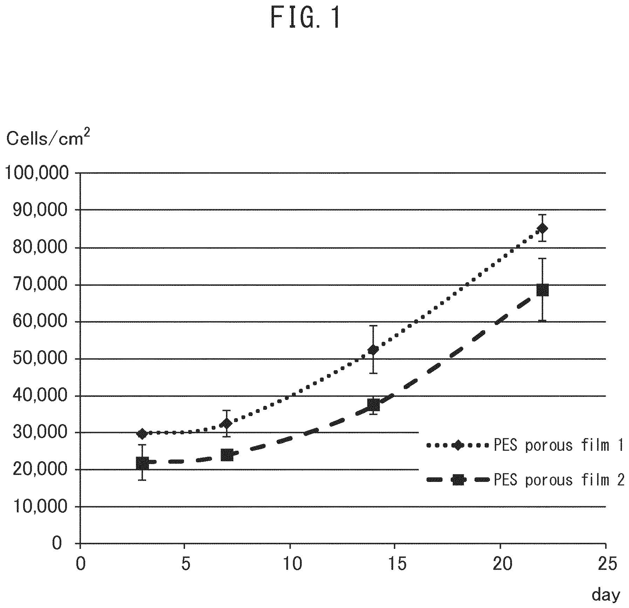

0.5 ml of cell culture medium was added to a sterilized 2 cm $\times$ 2 cm square vessel (Thermo Fisher Scientific, cat.103). Sterilized 1.4 cm-square porous PES films 1 and 2 were placed in the vessel with a mesh-structured surface A facing up, $4\times10^4$ human skin fibroblasts were seeded per porous PES film. Culturing was continued in a $CO_2$ incubator while regularly exchanging media twice a week. On days 3, 7, 15, and 22 after the start of culturing, cell count was measured using Cell Counting Kit-8 (Dojindo Molecular Technologies, Inc., hereinafter referred to as "CCK8"), and the cell growth behavior was observed. The results are depicted in FIG. 1. Stable cell growth was observed on the porous PES films.

Example 2

Long-term culture of human mesenchymal stem cells on porous PES films

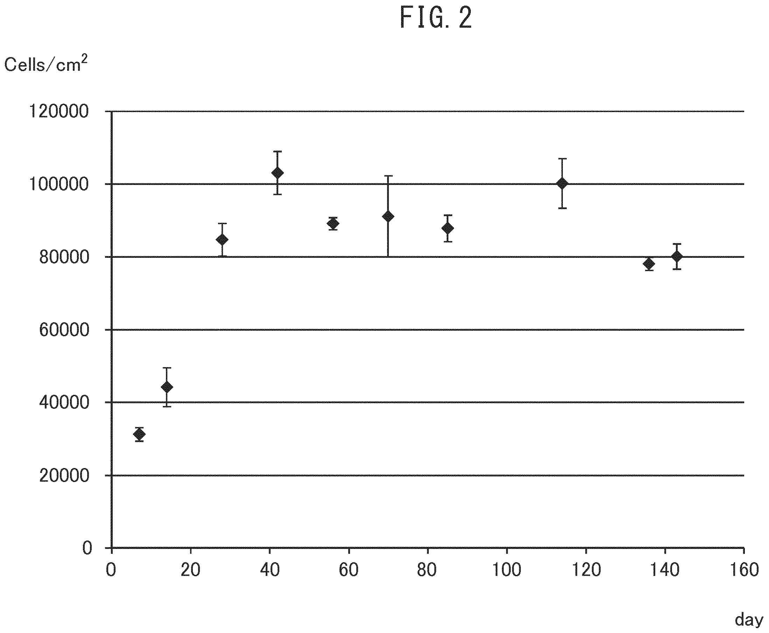

Human mesenchymal stem cells were seeded and cultured in a type I collagen-coated dish (IWAKI, mouth internal diameter: 6 cm), and then peeled off by trypsin treatment, thereby preparing a cell suspension. 0.5 ml of cell culture medium (GIBCO, DMEM+FBS10%) was added to a sterilized 2 cm $\times$ 2 cm square vessel (Thermo Fisher Scientific, cat.103). The sterilized 1.4 cm-square porous PES film 1 prepared in Example 1 was placed in the vessel with the mesh-structured surface A facing up, and then $4 \times 10^4$ human mesenchymal stem cells were added to the top of the film per porous PES film. Culturing was continued in a $CO_2$ incubator while regularly exchanging media twice a week. After the start of culturing, cells were cultured for 143 days while regularly measuring cell count using CCK8. The results are depicted in FIG. 2. On the porous PES film 1, stable growth of mesenchymal stem cells was observed. Hereinafter, the above way of culturing using the porous PES films is referred to as "member culture", and the thus obtained cell samples are referred to as "member culture cell samples".

Example 3

Induction of human mesenchymal stem cells cultured on porous PES films into fat cells

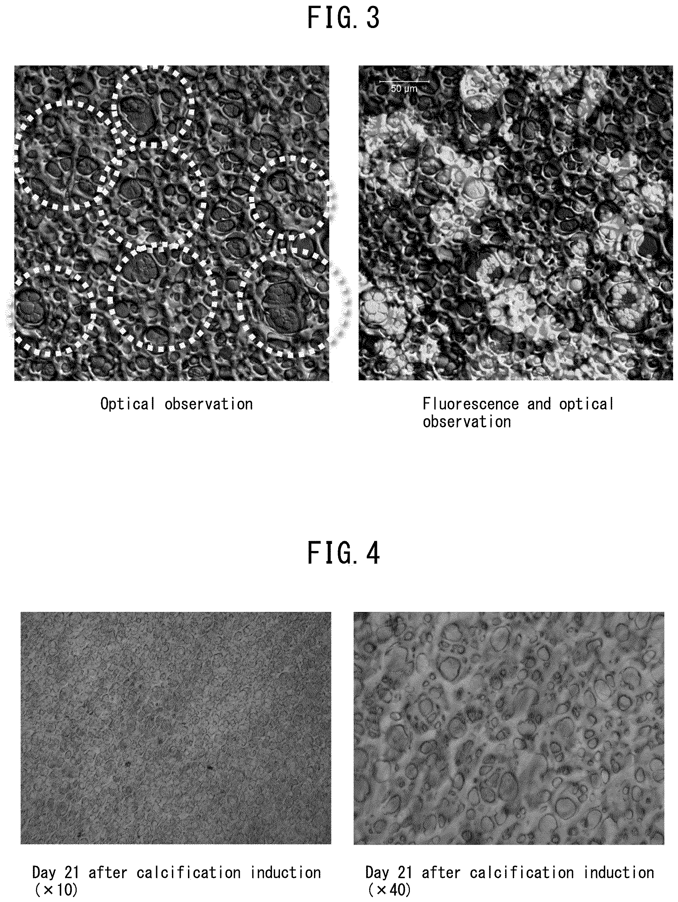

On day 99 after the start of culturing in Example 2, the porous PES film on which cells had survived and grown was transferred to a fat cell-inducing medium (PromoCell, C-28016), and then cells were cultured for 21 days. After 21 days of induction into fat cells, the porous PES film on which cells had survived and grown was fixed with formalin, and then fat droplets were stained by fluorescent staining with BODIPY. The results of optical observation, as well as optical and fluorescence observation of the same field of view using a confocal laser scanning microscope are depicted in FIG. 3. Because of a property, high visibility, of the porous PES film, the presence of fat droplets could be visually confirmed easily even optically. Even after long-term culture on the porous PES film, mesenchymal stem cells were found to maintain their induction property.

Example 4

Induction of human mesenchymal stem cells cultured on porous PES films into osteoblasts and mineralization induction

On day 99 after the start of culturing in Example 2, the porous PES film on which cells had survived and grown was transferred to an osteoblast differentiation-inducing medium (PromoCell, C-28013), and then cells were cultured for 21 days. Subsequently, mineralization induction was performed for 21 days in osteoblast mineralization medium (PromoCell, C-27020). After completion of mineralization induction, cells were fixed with methanol at a low temperature, and then stained using a calcified nodule staining kit (Cosmo Bio Co., Ltd.). Optical microscopy was performed to observe mineralized sites. Sites changed to red were observed, confirming the progress of mineralization (FIG. 4). Similar to fat cells, mesenchymal stem cells were confirmed to have also a property of being inducible to osteoblasts. High visibility of the porous PES film also contributes to improvement in observation.

Example 5

Genetic analysis of human mesenchymal stem cells cultured long-term on porous PES films

1. Preparation of samples

A member culture cell sample on day 139 after the start of culturing in Example 2 was used as a sample for genetic analysis. Moreover, under the same conditions as in Example 2 except for using no porous PES film, human mesenchymal stem cells were cultured for 7 days in a type I collagen-coated dish (IWAKI). The cultured cells were used as a sample for genetic analysis. The above culturing performed using no porous PES film is hereinafter referred to as "normal culture", and the thus obtained cell sample is referred to as a "normal culture cell sample".

2. Genetic analysis

The thus obtained samples were subjected to genetic analysis by the following procedures.

(1) RNA extraction

RNA was extracted using an RNeasy Plus micro Kit (Qiagen) according to protocols attached to the kit. RNA was extracted using 30 $\mu$L of Nuclease Free Water, and then genomic DNA was denatured with DNase using a TURBO DNA free Kit (Life Technologies). After denaturation, the concentration of the RNA solution was measured using Nano Drop 2000 (Thermo Fishier Scientific).

(2) cDNA synthesis

The RNA solution after measurement of the concentration was prepared so that it had a concentration of 12.5 ng/$\mu$L, and then cDNA synthesis was performed using 100 ng of the resultant as a template. For cDNA synthesis, SuperScript (trademark) III First-Strand Synthesis System for RT-PCR (Life Technologies) was used. The concentration of the cDNA solution was measured using Nano Drop 2000.

(3) q-PCR

The cDNA solution was prepared to have a concentration of 200 ng/$\mu$L, measurement was performed by real-time PCR using 200 ng of the solution as a template. PCR was performed with CFX connect (Bio-Rad), and SsoAdvanced (trademark) Universal SYBR Green Supermix (Bio-Rad) was used as a reagent. Expression levels of markers positive for mesenchymal stem cells (CD166, CD44, CD105, CD146, CD90, CD106, CD29, CD71), and markers negative for mesenchymal stem cells (CD19, CD45, CD31, CD18, CD56, CD34, CD14, CD80, CD40, CD86) were measured, where beta-Actin was used as an internal standard gene.

(4) Measured data analysis

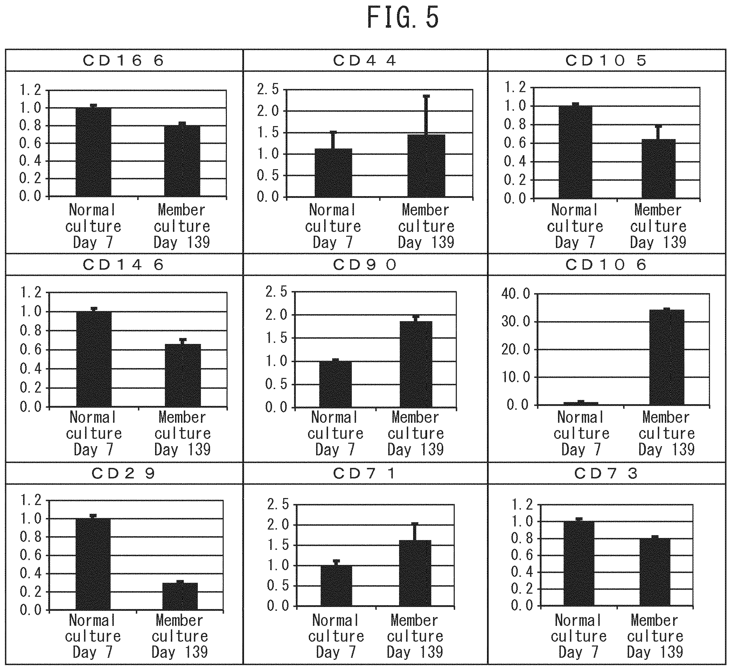

The Ct value of beta-Actin measured as the internal standard gene was subtracted from the Ct value of each gene found by measurement to calculate a relative expression level for comparison. Moreover, for comparison of changes over time in gene expression between normal culture cell samples and member culture cell samples, each change relative to the expression level of a cell sample from 7 days of normal culture designated as 1 was calculated and the results were compared. Results of the expression levels of the positive markers are depicted in FIG. 5. Further, the expression levels of the negative markers were all found to be low levels. It was confirmed by the gene expression levels that the properties of stem cells were maintained even after long-term culture using the porous PES film.

Example 6

Culture of human osteoblasts using porous PES film

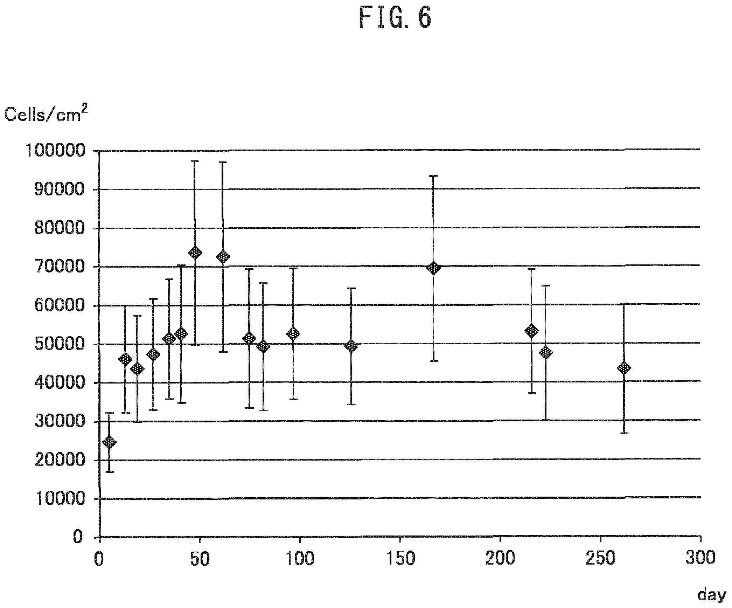

1 ml of osteoblast growth medium (PromoCell, C-27001) was added to a sterilized 2 cm $\times$ 2 cm square vessel (Thermo Fisher Scientific, cat.103), and then the sterilized 1.4 cm-square porous PES film 1 (prepared in Example 1) was dipped in the medium. $4\times10^4$ human osteoblasts (PromoCell) were seeded per porous PES film, and then continuously cultured in a $CO_2$ incubator. Twice-a-week medium exchange (1 ml) was continued, and cell count was regularly measured using CCK8. The results are depicted in FIG. 6. Even when long-term culture was performed, stable and high-density proliferation and growth of human osteoblasts were observed.

Example 7

Mineralization induction of human osteoblasts cultured on porous PES film

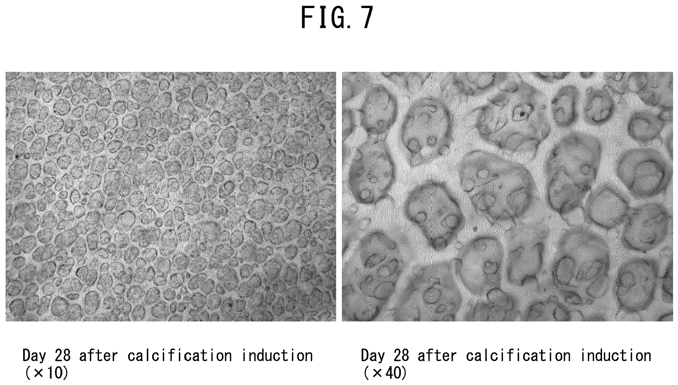

On day 224 after the start of culturing of Example 6, mineralization induction was performed using an osteoblast mineralization medium (PromoCell, C-27020) instead of the above medium. After 28 days, cells were stained using a calcified nodule staining kit (Cosmo Bio Co., Ltd.). Optical microscopy was performed to observe mineralized sites. Sites changed to red were observed, confirming the progress of mineralization (FIG. 7). It was confirmed that even after long-term culture of human osteoblasts on the porous PES film, the properties of osteoblasts were maintained. High visibility of the porous PES film contributes to improvement in observation of sites changed to red.

Example 8

Culturing of CHO DP-12 cells using porous PES films

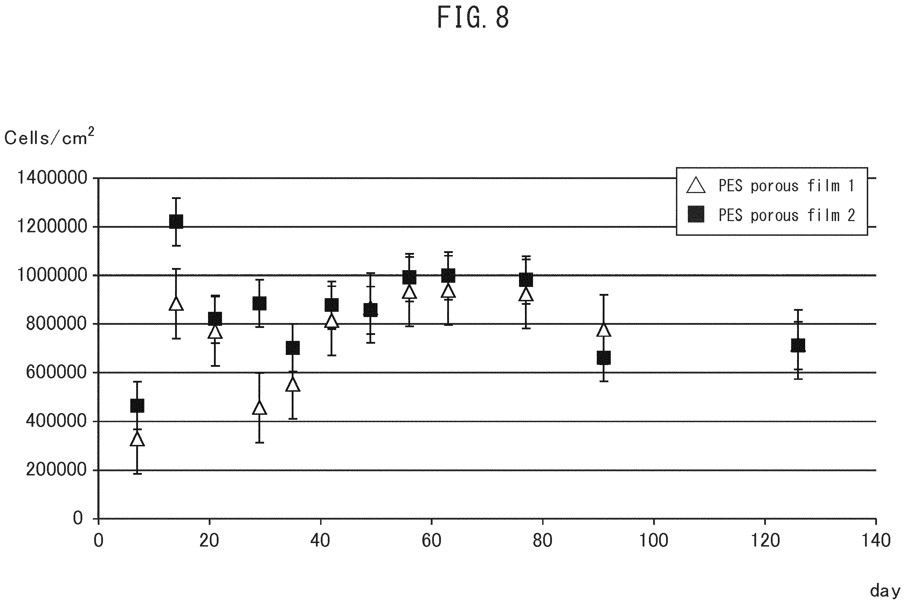

Anti-human IL-8 antibody-producing CHO DP-12 cells (ATCC CRL-12445) were seeded and cultured in a dish (mouth internal diameter: 10 cm), and then peeled off by trypsin treatment, thereby preparing a cell suspension. 0.5 ml of cell culture medium (IMDM, 2%FBS) was added to a sterilized 2 cm $\times$ 2 cm square vessel (Thermo Fisher Scientific, cat.103). The sterilized 1.4 cm-square porous PES films 1 and 2 (prepared in Example 1) were placed in a vessel with the mesh-structured surface A facing up, and then $4\times10^4$ CHO DP-12 cells were seeded per porous PES film. Cells were continuously cultured in a $CO_2$ incubator, while regularly exchanging media twice a week. After the start of culturing, cells were cultured for 143 days while regularly measuring cell count using CCK8. The results are depicted in FIG. 8. Stable proliferation and growth of CHO DP-12 were observed.

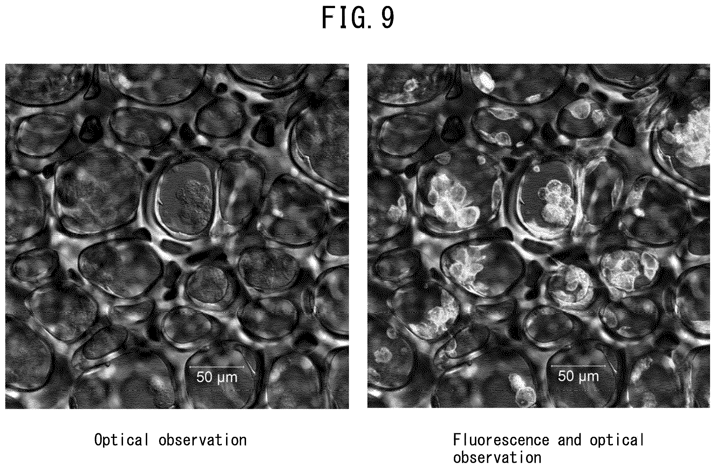

On day 143, the porous PES film 2 on which cells had grown was fixed, and then stained with Alexa Fluor (registered trademark) 488 Phalloidin, CellMask Orange Plasma Membrane Stain, and DAPI. The results of optical observation and optical and fluorescence observation of the same field of view using a confocal laser scanning microscope are depicted in FIG. 9. Because of a property of the porous PES film, high visibility, the presence of cells could be easily confirmed visually.

INDUSTRIAL APPLICABILITY

The method of the present invention can be used appropriately for culturing cells conveniently, efficiently, and stably. In particular, the porous polyethersulfone film is useful in that it has a low degree of pigmentation and thus have good visibility, and enables visual confirmation of the seeding of, and the adhesion and survival behavior of cells, etc. Furthermore, the porous polyethersulfone film is also useful in that it has a low degree of pigmentation, and thus is excellent in designability.

**Claims**

1.  A method for preparing cells, the method comprising:
    applying cells to a porous polyethersulfone film and culturing them.

2.  The method according to claim 1, wherein the porous polyethersulfone film is a three-layer structure porous polyethersulfone film having a surface layer A and a surface layer B, the surface layers having a plurality of pores, and a macrovoid layer sandwiched between the surface layers A and B;

    wherein an average pore diameter of the pores present in the surface layer A is smaller than an average pore diameter of the pores present in the surface layer B;
    wherein the macrovoid layer has a partition wall bonded to the surface layers A and B, and a plurality of macrovoids surrounded by the partition wall and the surface layers A and B; and
    wherein the pores in the surface layers A and B communicate with the macrovoids.

3.  The method according to claim 1 or 2, the method comprising the step of:
    seeding cells on the surface of the porous polyethersulfone film.

4.  The method according to claim 1 or 2, the method comprising the steps of:

    placing a cell suspension on the dried surface of the porous polyethersulfone film;
    allowing the porous polyethersulfone film to stand, or moving the porous polyethersulfone film to promote efflux of liquid, or stimulating a part of the surface to cause absorption of the cell suspension into the film; and
    retaining cells in the cell suspension inside the porous polyethersulfone film, and allowing water to flow out.

5.  The method according to claim 1 or 2, the method comprising the steps of:

    wetting one or both sides of the porous polyethersulfone film with a cell culture medium or a sterilized liquid;
    loading a cell suspension into the wetted porous polyethersulfone film; and
    retaining cells in the cell suspension inside the film, and allowing water to flow out.

6.  The method according to claim 5, wherein living cells remain within the porous polyethersulfone film, and dead cells flows out with the water.

7.  The method according to claim 5 or 6, wherein the sterilized liquid is a sterile water or a sterilized buffer solution.

8.  The method according to any one of claims 1 to 7, the method comprising the step of:
    placing a cell culture medium, cells, and one or more of the porous polyethersulfone films in a cell culture vessel, wherein the porous polyethersulfone films are in a suspended state in the cell culture medium.

9.  The method according to claim 8, **characterized in that** two or more pieces of the porous polyethersulfone films are used.

10. The method according to claim 8 or 9, wherein the cells spontaneously adhere to the porous polyethersulfone film.

11. The method according to any one of claims 1 to 7, wherein the porous polyethersulfone film is

i) folded,
ii) wound into a roll-like shape,
iii) connected as sheets or pieces with a filamentous structure, or
iv) bound into a rope-like shape,

and suspended or fixed in a cell culture medium in a cell culture vessel.

12. The method according to claim 11, wherein cells spontaneously adhere to the porous polyethersulfone film.

13. The method according to claim 1 or 2, the method comprising using two or more porous polyethersulfone films are layered either above and below or left and right in the cell culture medium.

14. The method according to claim 1 or 2, wherein two or more of the methods according to any one of claims 3 to 13 are conducted in combination.

15. The method according to any one of claims 1 to 14, wherein cells grow and proliferate on the surface and the inside of a porous polyethersulfone film.

16. The method according to any one of claims 1 to 15, wherein the cells are selected from the group consisting of animal cells, insect cells, plant cells, yeasts and bacteria.

17. The method according to claim 16, wherein the animal cells are cells derived from an animal belonging to the vertebrate phylum.

18. The method according to claim 16, wherein the bacteria are selected from the group consisting of lactic acid bacteria, Escherichia coli, Bacillus subtilis and cyanobacteria.

19. The method according to any one of claims 1 to 15, wherein the cells are selected from the group consisting of pluripotent stem cells, tissue stem cells, somatic cells and germ cells.

20. The method according to any one of claims 1 to 15, wherein the cells are selected from the group consisting of sarcoma cells, established cells and transformed cells.

21. A cell culture apparatus for use in a method for preparing cells according to any one of claims 1 to 20, the apparatus comprising a porous polyethersulfone film.

22. The cell culture apparatus according to claim 21, wherein two or more porous polyethersulfone films are layered either above and below or left and right.

23. A kit for use in a method for preparing cells according to any one of claims 1 to 20, the kit comprising a porous polyethersulfone film.

# FIG. 1

FIG. 2

Cells/cm$^2$

120000
100000
80000
60000
40000
20000
0

0 20 40 60 80 100 120 140 160

day

# FIG. 3

Optical observation

Fluorescence and optical
observation

# FIG. 4

Day 21 after calcification induction
(×10)

Day 21 after calcification induction
(×40)

FIG. 5

FIG. 6

EP 3 489 349 A1

# FIG. 7

Day 28 after calcification induction
(×10)

Day 28 after calcification induction
(×40)

FIG.8

FIG. 9

Fluorescence and optical observation

Optical observation

## INTERNATIONAL SEARCH REPORT

International application No.

PCT/JP2017/026938

### A. CLASSIFICATION OF SUBJECT MATTER

*C12N1/00*(2006.01)i, *C12M1/00*(2006.01)i, *C12M3/00*(2006.01)i, *C12N5/00* (2006.01)i

According to International Patent Classification (IPC) or to both national classification and IPC

### B. FIELDS SEARCHED

Minimum documentation searched (classification system followed by classification symbols)
C12N1/00-7/08, C12M1/00-3/10

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
JSTPlus/JMEDPlus/JST7580(JDreamIII), CAplus/MEDLINE/EMBASE/BIOSIS(STN), DWPI(Derwent Innovation)

### C. DOCUMENTS CONSIDERED TO BE RELEVANT

| Category* | Citation of document, with indication, where appropriate, of the relevant passages | Relevant to claim No. |
|---|---|---|
| X<br>Y | BARTOLO L.D., et al., Polyethersulfone membrane biohybrid system using pig hepatocytes: Effect of diclofenac on cell biotransformation and synthetic functions, J. Membr. Sci., 2006, Vol.278, p.133-143, ISBN 0376-7388, particularly, Abstract, page 134, left column, line 41 to right column, line 12, page 137, left column, lines 9 to 10, Fig. 1-2 | 1,3,15-17,19<br>1-23 |
| Y | WO 2015/012415 A1 (Ube Industries, Ltd.), 29 January 2015 (29.01.2015), claims; paragraphs [0093] to [0095] & US 2016/0168560 A1 claims; paragraphs [0137] to [0139] | 1-23 |

| ☒ | Further documents are listed in the continuation of Box C. | ☐ | See patent family annex. |
|---|---|---|---|

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered    to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 16 October 2017 (16.10.17) | 24 October 2017 (24.10.17) |

| Name and mailing address of the ISA/<br>Japan Patent Office<br>3-4-3,Kasumigaseki,Chiyoda-ku,<br>Tokyo 100-8915,Japan | Authorized officer<br><br><br>Telephone No. |
|---|---|

Form PCT/ISA/210 (second sheet) (January 2015)

**INTERNATIONAL SEARCH REPORT**

| International application No. |
| --- |
| PCT/JP2017/026938 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | JP 2009-538617 A  (CHA Biotech Co., Ltd.),<br>12 November 2009 (12.11.2009),<br>claims<br>& WO 2007/139357 A1<br>claims | 1-23 |
| Y | JP 2012-503688 A  (Gambro Lundia AB.),<br>09 February 2012 (09.02.2012),<br>claims<br>& WO 2010/034466 A1<br>claims | 1-23 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015012415 A
- WO 2009123349 A

- JP 2009057041 W